(19) Europäisches Patentamt
European Patent Office
Office européen des brevets

(11) Numéro de publication: 0 323 304
A1

# (12) DEMANDE DE BREVET EUROPEEN

(21) Numéro de dépôt: 88403183.2

(22) Date de dépôt: 14.12.88

(51) Int. Cl.⁴: C 01 G 49/00
B 01 J 23/00

(30) Priorité: 16.12.87 FR 8717575

(43) Date de publication de la demande:
05.07.89 Bulletin 89/27

(84) Etats contractants désignés:
AT BE CH DE ES FR GB GR IT LI LU NL SE

(71) Demandeur: ATOCHEM
4 & 8, Cours Michelet La Défense 10
F-92800 Puteaux (FR)

(72) Inventeur: Rousset, Abel
16, Rue Jean Moulin
F-31520 Ramonville Saint-Agne (FR)

Maachi, Belaid
90, Rue Zractouni
Segangan-Nador (MA)

Gilot, Bernard
61, Rue Boyssonne
F-31400 Toulouse (FR)

Gougeon, Michel
28, Rue Henri de Sahuqué
F-31400 Toulouse (FR)

(74) Mandataire: Leboulenger, Jean et al
ATOCHEM Département Propriété Industrielle
F-92091 Paris la Défense 10 Cédex 42 (FR)

## (54) Composition à base d'un oxyde de structure spinelle, son application comme catalyseur et procédé pour l'obtenir.

(57) L'invention concerne une composition qui comprend un oxyde de structure spinelle du type γ $Fe_2O_3$ - $Fe_3O_4$ substitué par des métaux trivalents et monovalents ou dopés par leurs dérivés ne faisant pas partie de la structure spinelle.

L'invention concerne plus particulièrement un oxyde de fer, aluminium et potassium et son usage comme catalyseur de déshydrogénation de l'éthylbenzène en styrène. Cette composition est préparée par décomposition thermique d'oxalates mixtes de fer, aluminium et potassium en présence d'air suivie d'une réduction en présence de vapeur d'eau et d'hydrogène à une température inférieure à 400°C, puis d'une oxydation.

L'invention concerne aussi la préparation d'oxydes de structure alumine β.

EP 0 323 304 A1

## Description

### COMPOSITION A BASE D'UN OXYDE DE STRUCTURE SPINELLE, SON APPLICATION COMME CATALYSEUR ET PROCEDE POUR L'OBTENIR

La présente invention concerne une composition qui comprend un oxyde de fer de structure spinelle contenant aussi des métaux trivalents et monovalents faisant partie de la structure spinelle. Cette composition est particulièrement utile comme catalyseur de déshydrogénation, notamment de l'éthylbenzène en styrène. Cette composition peut être obtenue par décomposition d'un sel organique mixte de fer desdits métaux trivalents et monovalents suivie d'une réduction en présence d'hydrogène et de vapeur d'eau et éventuellement d'une oxydation.

La déshydrogénation des hydrocarbures est une réaction très souvent effectuée dans l'industrie, par exemple pour préparer du butadiène à partir de butène ou du styrène à partir d'éthylbenzène. Le brevet FR 2.506.294 décrit un catalyseur de déshydrogénation formé d'oxyde de fer à structure spinelle contenant des métaux trivalents tels que l'aluminium, le chrome, des métaux divalents tels que le calcium, le manganèse, tous ces métaux étant dans la structure spinelle. La structure spinelle contient aussi du lithium et peut contenir du sodium, de l'argent ou du cuivre. Ce catalyseur est amélioré par addition d'oxydes de métaux alcalins et d'oxyde de vanadium hors de la structure de spinelle. La surface d'un tel catalyseur ne dépasse pas $2 m^2/g$.

La demande de brevet européen EP 177.832 décrit des catalyseurs de déshydrogénation formés par calcination d'oxydes de fer, de chrome, de potassium et de magnesium.

Un catalyseur de faible surface est souvent peu actif c'est-à-dire qu'il en faut une grande quantité pour assurer une production horaire donnée de produit déshydrogéné et les oxydes de métaux qui ne font pas partie de la structure spinelle ont tendance à migrer hors du cata et à disparaitre.

On a maintenant trouvé un nouvel oxyde de fer à structure spinelle qui peut convenir comme catalyseur de déshydrogénation.

La présente invention est une composition à base d'un oxyde ayant la structure de spinelle lacunaire, caractérisée en ce que sa surface spécifique est supérieure à $2 m^2/g$ et en ce qu'elle comprend :

a) au moins un oxyde de structure spinelle lacunaire du type $\gamma\ Fe_2O_3$ et de formule :

$$\left[\left(Fe^{3+}_{1-x}\ M^{3+}_{x}\right)_{8-t}\ \square_{1-2t}\ Q^{+}_{3t}\right] O^{2-}_{12}$$

dans laquelle M désigne un métal trivalent ou un mélange quelconque de ces métaux, Q désigne un métal monovalent ou un mélange quelconque de ces métaux, x est compris entre 0 et 2/3, t est compris entre 0 et 0,5

b) éventuellement au moins un dérivé d'un ou plusieurs métaux Q ne faisant pas partie de la structure spinelle et pouvant être différents du métal Q de cette structure,

Le terme spinelle désigne les membres d'un group d'oxydes multiples ayant la même structure cristalline que le spinelle minéral spécifique $MgAl_2O_4$. Dans la formule ci-dessus le signe " □ " désigne une lacune dans la structure spinelle.

La présente invention concerne aussi une composition identique à la précédente sauf que l'oxyde de structure spinelle lacunaire du type $\gamma\ Fe_2O_3$ est remplacé par un oxyde de structure spinelle du type $Fe_3O_4$ dont la formule dérive de la précédente.

Quand on dit que la formule de l'oxyde dérive de la précédente, on veut dire que la formule brute contient les mêmes métaux en dehors de l'oxygène.

La présente invention concerne aussi toute composition intermédiaire entre les deux précédentes, c'est-à-dire que l'oxyde de structure spinelle contenu dans la composition est une solution solide d'un oxyde de structure spinelle de type $\gamma\ Fe_2O_3$ et d'un oxyde de structure spinelle de type $Fe_3O_4$, les deux oxydes ayant la même formule brute en dehors de l'oxygène.

Bien qu'on puisse utiliser tout métal trivalent M ou combinaison de ces métaux y compris les terres rares c'est-à-dire les éléments du numéro atomique 57 au numéro atomique 71 de la classification périodique, on préfère le chrome, l'aluminium ou leurs mélanges. Le métal monovalent Q peut être par exemple choisi parmi les alcalins, l'argent et le cuivre. On peut utiliser un ou plusieurs métaux Q. On utilise avantageusement les alcalins et de préférence le potassium.

Bien que x puisse être compris entre 0 et 2/3 on préfère les compositions dans lesquelles x est inférieure à 0,2.

Bien que t puisse prendre toute valeur entre 0 et 0,5 on préfère les compositions dans lesquelles t est inférieur à 0,25.

La surface spécifique est celle mesurée par la méthode BET (BRUNAUER, EMETT et TELLER, J. Amer.

Chem. Soc., 60, 1938, 309). On utilise les compositions dont la surface spécifique est comprise entre 2 et 100 $m^2/g$ et de préférence entre 3 et 30 $m^2/g$.

Parmi les compositions ou Q est le potassium on préfère celles dans lesquelles t est compris entre 0,02 et 0,15.

Parmi les compositions ou M est l'aluminium on préfère celles dans lesquelles x est compris entre 0,05 et 0,2.

Les compositions selon l'invention peuvent contenir éventuellement un ou plusieurs dérivés de métaux choisis parmi les métaux Q et ne faisant pas partie de la structure spinelle. Ce ou ces métaux peuvent être différents du ou des métaux Q inclus dans la structure spinelle. Par exemple on peut avoir du sodium et du lithium dans la structure spinelle et du potassium en dehors de la structure spinelle ou l'inverse. On préfère les compositions dans lesquelles on a du potassium dans la structure spinelle et en dehors de cette structure. Avantageusement les dérivés de ces métaux sont des oxydes. De préference on utilise l'oxyde de potassium.

Au cours de l'utilisation des compositions selon l'invention, les oxydes hors de la structure spinelle peuvent être transformés, en partie ou en totalité, en d'autres dérivés, par exemple en carbonate et bicarbonate selon la teneur en oxygène de la phase gazeuse où sont les compositions et si du carbone vient au contact des compositions.

De même selon la phase gazeuse dans laquelle est placée la composition selon l'invention on peut avoir une évolution de la répartition des oxydes de type $\gamma$ $Fe_2O_3$ et $Fe_3O_4$.

La présente invention concerne aussi un procédé de déshydrogénation d'un produit hydrocarboné caractérisé en ce qu'on utilise comme catalyseur une composition selon l'invention.

Le produit hydrocarboné est tout produit susceptible de perdre deux atomes d'hydrogène pour former une double liaison carbone-carbone. Ce produit hydrocarboné de départ peut aussi posséder une double liaison et le procédé de l'invention permet alors de créer une deuxième double liaison dans la molécule. Le procédé de l'invention est particulièrement utile pour déshydrogéner l'éthylbenzène en styrène.

La présente invention concerne aussi un procédé de fabrication de ces compositions.

Le procédé est caractérisé en ce que :

a) on décompose des sels organiques mixtes des métaux M et Q et à base de fer en présence d'air ou d'un gaz contenant de l'oxygène jusqu'à obtenir des oxydes des métaux M, Q et du fer.

b) on effectue une réduction desdits oxydes métalliques jusqu'à obtenir une composition comprenant lesdits oxydes métalliques sous forme de spinelle du type $Fe_3O_4$.

c) on effectue éventuellement une oxydation totale ou partielle des compositions obtenues en b) jusqu'à obtenir une composition comprenant lesdits oxydes métalliques sous forme de spinelle du type $Fe_3O_4$ et lesdits oxydes métalliques sous forme de spinelle lacunaire du type $\gamma$ $Fe_2O_3$ dont la formule dérive des précédentes, les deux oxydes du type $\gamma$ $Fe_2O_3$ et $Fe_3O_4$ forment une solution solide.

L'expression "sel mixte" désigne le sel au sein duquel les métaux entrant dans la composition de l'oxyde sont déjà combinés à l'échelle atomique par échange de liaisons ou par intercroissance entre les sels simples, étant donné l'isomorphie de structure entre les sels simples de ces métaux.

Les sels mixtes des métaux M, Q et du fer peuvent être des acétates, formiates, citrates, oxalates ou lactates et en général tout produit formant des sels des métaux M, Q et du fer et qui sont mutuellement solubles dans l'eau ou les alcools ou les cétones ou les éthers.

On préfère cependant utiliser les sels provenant de l'acide oxalique.

Lors de la décomposition des sels, la vitesse de montée en température est de préférence inférieure à 300°C/h pour éviter une réduction transitoire en fer métal et la démixtion des oxydes résultants.

Bien qu'on puisse effectuer l'étape a) dans une large plage de température, on préfère se placer entre 250 et 500°C.

La réduction de l'étape b) s'effectue avantageusement en présence d'un mélange essentiellement formé de vapeur d'eau et d'hydrogène ou d'un mélange d'oxyde de carbone et de dioxyde de carbone.

On préfère effectuer l'étape b) à une température comprise entre 250° et 800°C.

Un autre mode préféré de l'étape b) consiste à effectuer la réduction entre 350° et 380°C en présence d'un mélange de vapeur d'eau et d'hydrogène.

Pour préparer les compositions selon l'invention contenant différents métaux dans les proportions souhaitées, il suffit de préparer des sels mixtes ayant ces métaux dans les mêmes proportions.

Une variante du procédé de l'invention permet de préparer des ferrites de structure alumine $\beta$. Alors que dans le procédé qu'on vient de décrire on obtient à l'issue de l'étape a) des oxydes amorphes, la demanderesse a découvert qu'en effectuant l'étape a) à haute température on obtient des oxydes de structure alumine $\beta$.

L'invention est un procédé de préparation d'oxydes de formule brute :

$K_{1+z}\ Fe_{11-x-y}\ Al_x\ Cr_y\ O_{17}$

et de structure alumine $\beta$ dans laquelle :

- x + y est compris entre 0 et 11
- x et y sont positifs
- z est compris entre 0 et 2 et représente l'écart à la stoechiométrie,

caractérisée en ce qu'on décompose des sels organiques mixtes de Fe, Al, Cr, K à une température au moins égale à 550°C jusqu'à obtenir l'oxyde de structure alumine $\beta$.

L'invention est particulièrement utile pour la préparation d'oxydes de surface spécifique (surface BET)

jusqu'à 100 $m^2/g$ et avantageusement comprise entre 10 et 60 $m^2/g$. On utilise avantageusement les mêmes sels mixtes que précédemment, et on préfère les oxalates. Il suffit de préparer les sels mixtes en utilisant les proportions des métaux de la formule brute recherchée.

La température de décomposition est habituellement inférieure à 1200° C et de préférence comprise entre 700 et 1000° C et la durée de cette décomposition est de l'ordre de 30 minutes à 3 heures.

Les exemples suivant illustrent l'invention sans la limiter.

Les exemples 1 à 7 illustrent les oxydes de structure spinelle :
$\gamma$ $Fe_2O_3$ - $Fe_3O_4$,
l'exemple 8 illustre l'oxyde de structure alumine $\beta$.

## 1 - PREPARATION DES SELS MIXTES

Les sels mixtes de formule générale :
$[(NH_4)_{1-x} K_x]_3 (Fe_{1-y-y'} Al_y Cr_{y'}) (C_2O_4)_3 3H_2O$ dans lesquels x et y + y' sont compris entre 0 et 1, ont été préparés par syncristallisation des oxalates simples choisis parmi $(NH_4)_3$, $Fe(C_2O_4)_3$, $3H_2O$ ; $K_3 Fe(C_2O_4)_3$, $3H_2O$ ; $(NH_4)_3 Al(C_2O_4)_3$, $3H_2O$ ; $K_3 Al(C_2O_4)_3$, $3H_2O$ ; $(NH_4)_3 Cr(C_2O_4)_3$, $3H_2O$ et $K_3 Cr(C_2O_4)_3$, $3H_2O$.

### a - Oxalates mixtes : mode opératoire 1

a grammes d'oxalate fer-ammonium, b grammes d'oxalate de fer-potassium, c grammes d'oxalate d'aluminium-ammonium, d grammes d'oxalate de potassium-aluminium, e grammes d'oxalate de chrome-ammonium et f grammes d'oxalate de chrome-potassium sont dissous dans 2 l d'eau désionisée. Le mélange est alors porté sous agitation à une température de 70° C pour concentrer la solution. A l'ammorce de la recristallisation, le chauffage est arrêté et la solution refroidie. La syncristallisation des oxalates est poursuivie au bain de sable à 40° C jusqu'à évaporation totale de la solution. Les valeurs de a, b, c, d, e et f pour la préparation de 100 g de chacun des différents oxydes, ont été reportées au tableau I.

### b - Oxalates simples non disponibles commercialement

. Mode opératoire 2 :

L'oxalate $(NH_4)_3 M(C_2O_4)_3$, $3H_2O$ est préparé par l'addition progressive d'hydroxyde fraichement préparé, du métal trivalent M, à une solution aqeuse d'acide oxalique et d'oxalate d'ammonium maintenue à 50° C. Les proportions respectives d'hydroxyde, d'acide oxalique et d'oxalate d'ammonium à mettre en oeuvre sont celles correspondant à la stoechiométrie de la réaction. La solution obtenue est ensuite concentrée pour initier la cristallisation. Cette dernière est réalisée au bain de sable à 40° C jusqu'à évaporation complète de la solution.

. Mode opératoire 3 :

L'oxalate $K_3M(C_2O_4)_3$, $3H_2O$ est préparé suivant le mode opératoire 2, l'oxalate d'ammonium étant remplacé par l'oxalate de potassium.

## 2 - PREPARATIONS DES OXYDES

. Mode opératoire 4 : exemple 1

Le sel mixte fer-ammonium-potassium est décomposé par pyrolyse à l'air à 450° C pendant 1 heure. La vitesse de montée en température à 450° C est de 150° C/h. Les oxydes alors obtenus, de grande surface spécifique sont ensuite réduits à 350° C pendant 4 heures par un mélange gazeux 70 % hydrogène - 30 % vapeur d'eau. L'oxyde résultant est majoritairement composé d'une phase spinelle de type $Fe_3O_4$.

. Mode opératoire 5 : exemples 2 et 3

La pyrolyse du sel fer-ammonium-potassium à 450° C pendant 1 heure et la réduction à 350° C pendant 4 heures sont effectuées suivant le mode opératoire 4. Elles sont suivies d'une oxydation à l'air à 400° C pendant 1 heure. La vitesse de montée en température à 400° C est de 150° C/h. L'oxyde obtenu se compose principalement d'une phase spinelle lacunaire de type $\gamma$-$Fe_2O_3$.

. Mode opératoire 6 : exemple 4

La pyrolyse du sel fer-ammonium-potassium à 450° C pendant 1 heure et la réduction à 350° C pendant 4 heures sont effectuées suivant le mode opératoire 4. Elles sont suivies d'une oxydation à l'air à 130° C pendant 2 heures. L'oxyde obtenu contient majoritairement une solution solide de composition intermédiaire entre la magnétite substituée et le spinelle lacunaire correspondant.

. Mode opératoire 7 : exemples 5, 6 et 7

Le sel mixte fer-aluminium-potassium-ammonium ou fer-chrome-potassium-ammonium est décomposé par pyrolyse à l'air à 400° C pendant 1 heure. La vitesse de montée en température à 400° C est de 150° C/h. Les oxydes alors obtenus sont amorphes. Ils sont ensuite réduits à 370° C pendant 5 heures par un mélange gazeux contenant 60 % d'hydrogène et 40 % de vapeur d'eau, trempés et finalement oxydés à 400° C pendant 1 heure avec une vitesse de montée en température à 400° C de 150° C/h. L'oxyde obtenu est

majoritairement composé d'une phase spinelle lacunaire.

. Mode opératoire 8 : exemple 8

Le sel fer-potassium-ammonium est pyrolysé à 950°C pendant 1 heure, la vitesse de montée en température est de 150°C/h. L'oxyde résultant, pour une teneur massique en potassium dans l'oxyde de 6 à 8 %, est un ferrite de potassium de type alumine β qui se forme dès 600°C.

3 - MESURE DE L'ACTIVITE CATALYTIQUE : REACTION DE DESHYDROGENATION DE L'ETHYLBENZENE

Les activités catalytiques des compositions selon l'invention ont été mesurées à 550°C à la pression atmosphérique pour une pression partielle d'éthylbenzène égale à 7,5 $10^3$ Pa et un rapport pression partielle de vapeur d'eau/pression partielle d'éthylbenzène égale à 20. Le débit d'éthylbenzène est de $10^{-5}$ moles par seconde et par gramme de catalyseur.

Les résultats figurent au tableau I sous la forme du rapport de l'activité massique de l'oxyde sur l'activité massique d'un catalyseur mesurée dans les mêmes conditions expérimentales.

Le catalyseur existant servant de référence est obtenu par calcination d'un mélange en poids de 93 % de $Fe_2O_3$, 5 % de $Cr_2O_3$ et 2 % de KOH.

TABLEAU I

| EXEMPLES | PRECURSEUR MIXTE a | b | c | d | COMPOSITION DE L'OXYDE Composition de la phase spinelle | % $K_2O$ 2* | Paramètre cristallin A° 3* | Surface spécifique $m^2/g$ 4* | Température d'oxydation °C 5* | Température de transformation 6* | Activité 7* |
|---|---|---|---|---|---|---|---|---|---|---|---|
| | en grammes | | | | | | | | | | |
| 2 | 517 | 8,38 | | | $K^+_{0,247}Fe^{3+}_{7,948}\square_{0,835}O^{2-}_{12}$ | ~0,6% | 8,35 | 23 | - | 720 | 12 |
| 1 | 517 | 8,38 | | | $K^+_{0,272}Fe^{3+}_{6,272}Fe^{2+}_{2,455}O^{2-}_{12}$ | ~0,6% | 8,40 | 24,5 | 210 | - | 12,5 |
| 4 | 517 | 8,38 | | | $K^+_{0,262}Fe^{3+}_{6,969}Fe^{2+}_{1,415}\square_{0,354}O^{2-}_{12}$ | ~0,6% | 8,375 | 24 | - | 720 | 2,5 |
| 3 | 456 | 33,5 | | | $K^+_{0,314}Fe^{3+}_{7,895}\square_{0,791}O^{2}_{12}$ | ~7,3 | 8,40 | 15 | - | 700 | 5,6 |
| 5 | 495 | | 10,64 | 15,98 | $K^+_{0,478}Fe^{3+}_{7,449}Al_{0,392}\square_{0,682}O^{2-}_{12}$ | ~1,3% | 8,33 | 33 | - | 735 | 6 |
| 6 | 296 | 25,1 | 263 | | $K^+_{0,423}Fe^{3+}_{4,165}Al^{3+}_{3,694}\square_{0,718}O^{2-}_{12}$ | ~3 % | 8,31 | 21 | - | 800 | 2,6 |
| | a | b | e | f | | | | | | | |
| 7 | 465 | | 39,46 | 15,03 | $K^+_{0,412}Fe^{3+}_{7,07}Cr^{3+}_{0,786}$ $_{0,725}O^{2-}_{12}$ | ~1 % | 8,34 | 30 | - | 725 | 10 |
| | a | b | c | d | Ferrite de potassium | | | | | | |
| 8 | 453 | | 19,7 | 31,97 | $K_{1,8}(Fe_{9,9}Al_{0,1})_{11}O_{17}$ | ~0 % | $\vec{a}$=5,92 $\vec{c}$=23,4 | 12,4 | - | - | 3 |

1* masses des oxalates simples mises en oeuvre, pour la préparation de 100 g d'oxyde.
2* % $K_2O$ hors de la phase spinelle par rapport à la masse totale d'oxyde (le plus souvent sous forme de carbonates $K_2CO_3$-3/2$H_2O$,$KHCO_3$).
3* de la phase spinelle.
4* de l'oxyde déterminée par la méthode B.E.T.
5* pour les magnétites, déterminée par analyse thermique différentielle vitesse de montée en température 10°C/mn.
6* transformation spinelle lacunaire - oxyde de structure corindon. ATD 10°C/mn.
7* rapport activité massique de l'oxyde/activité d'un catalyseur existant.

**Revendications**

1. Composition à base d'un oxyde ayant la structure de spinelle lacunaire, caractérisée en ce que sa surface spécifique est supérieure à 2 $m^2/g$ et en ce qu'elle comprend :

a) au moins un oxyde de structure spinelle lacunaire du type $\gamma\ Fe_2O_3$ et de formule :

$$\left[\left(Fe^{3+}_{1-x}\ M^{3+}_{x}\right)_{8-t}\ \square_{1-2t}\ Q^{+}_{3t}\right]O^{2-}_{12}$$

dans laquelle M désigne un métal trivalent ou un mélange quelconque de ces métaux, Q désigne un métal monovalent ou un mélange quelconque de ces métaux, x est compris entre 0 et 2/3, t est compris entre 0 et 0,5,

b) éventuellement au moins un dérivé d'un ou plusieurs métaux Q ne faisant pas partie de la structure spinelle et pouvant être différents du métal Q de cette structure,

2. Composition selon la revendication 1, caractérisée en ce que l'oxyde de structure spinelle lacunaire du type $\gamma\ Fe_2O_3$ est remplacé par un oxyde de structure spinelle du type $Fe_3O_4$ dont la formule dérive de la précédente.

3. Composition selon la revendication 1, caractérisé en ce que l'oxyde de structure spinelle lacunaire du type $\gamma\ Fe_2O_3$ est partiellement remplacé par un oxyde de structure dont la formule dérive de la précédente, les deux oxydes du type $\gamma\ Fe_2O_3$ et $Fe_3O_4$ formant une solution solide.

4. Composition selon l'une des revendications 1 à 3, caractérisée en ce que x est inférieur à 0,2.

5. Composition selon l'une des revendications 1 à 4, caractérisée en ce que t est inférieur à 0,25.

6. Composition selon l'une des revendications 1 à 5, caractérisée en ce que la surface spécifique est comprise entre 2 et 100 $m^2/g$.

7. Composition selon l'une des revendications 1 à 6, caractérisée en ce que M est de l'aluminium.

8. Composition selon la revendication 7, caractérisée en ce que x est de préférence compris entre 0,05 et 0,2.

9. Composition selon l'une des revendications 1 à 8, caractérisée en ce que Q est du potassium.

10. Composition selon la revendication 9, caractérisée en ce que t est de préférence compris entre 0,02 et 0,15.

11. Composition selon la revendication 9 ou 10, caractérisée en ce qu'elle comprend de l'oxyde de potassium ne faisant pas partie de la structure spinelle.

12. Procédé de déshydrogénation d'un produit hydrocarboné, caractérisé en ce qu'on utilise comme catalyseur une composition selon l'une des revendications 1 à 11.

13. Procédé selon la revendication 12, caractérisé en ce qu'on l'applique à la déshydrogénation de l'éthylbenzène en styrène.

14. Procédé de fabrication des compositions selon l'une des revendications 1 à 11, caractérisé en ce que :

a) on décompose des sels organiques mixtes des métaux M et Q et à base de fer en présence d'air ou d'un gaz contenant de l'oxygène jusqu'à obtenir des oxydes des métaux M, Q et du fer.

b) on effectue une réduction desdits oxydes métalliques jusqu'à obtenir une composition comprenant lesdits oxydes métalliques sous forme de spinelle du type $Fe_3O_4$.

c) on effectue éventuellement une oxydation totale ou partielle des compositions obtenues en b) jusqu'à obtenir une composition comprenant lesdits oxydes métalliques sous forme de spinelle du type $Fe_3O_4$ et lesdits oxydes métalliques sous forme de spinelle lacunaire du type $\gamma\ Fe_2O_3$ dont la formule dérive des précédentes, les deux oxydes du type $\gamma\ Fe_2O_3$ et $Fe_3O_4$ forment une solution solide.

15. Procédé selon la revendication 14 caractérisé en ce qu'on effectue l'étape a) entre 250 et 500° C.

16. Procédé selon la revendication 14 ou 15 caractérisé en ce qu'on effectue l'étape b) en présence d'un mélange essentiellement formé de vapeur d'eau et d'hydrogène ou d'oxyde de carbone et de dioxyde de carbone.

17. Procédé selon l'une des revendications 14 à 16 caractérisé en ce qu'on effectue l'étape b) entre 250 et 800° C.

18. Procédé selon l'une des revendications 14 à 17, caractérisé en ce que les sels organiques sont des oxalates.

19. Procédé selon l'une des revendications 14 à 18, caractérisé en ce que l'étape b) est effectuée entre

350 et 380° C en présence d'un mélange de vapeur d'eau et d'hydrogène.

20. Procédé de préparation d'oxydes de formule :

$K_{1+z}Fe_{11-x-y}Al_xCr_yO_{17}$

et de structure alumine β dans laquelle :

- x + y est compris entre 0 et 11
- x et y sont positifs
- z est compris entre 0 et 2 et représente l'écart à la stoechiométrie

caractérisé en ce qu'on décompose des sels organiques mixtes de Fe, Al, Cr, K à une température d'au moins 550° C jusqu'à obtenir l'oxyde de structure alumine β.

Office européen des brevets

# RAPPORT DE RECHERCHE EUROPEENNE

Numero de la demande

EP 88 40 3183

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | P. PASCAL: "Nouveau traite de chimie minerale", Tome XVII, 1963, page 659, Masson et Cie, Paris, FR<br>* Page 659, lignes 14-36 *<br>--- | 1,5,9 | C 01 G 49/00<br>B 01 J 23/00 |
| A | WO-A-8 702 173 (CNRS)<br>* Page 20, revendications 1-3; pages 21,22, revendications 10,15 * | 1 | |
| A | | 14,15, 18 | |
| A | * Page 3, paragraphe 4 *<br>--- | 11 | |
| A | FR-A-2 034 287 (A.C. ROUSSET)<br>* Page 1, lignes 31-39; page 2, lignes 1-17 * | 1 | |
| A | * Page 7, exemple 1 *<br>--- | 10,16-19 | |
| A | DE-B-2 627 454 (ROBERT BOSCH)<br>--- | | |
| A | FR-A-2 506 294 (SHELL FRANCAISE)<br>--- | | DOMAINES TECHNIQUES RECHERCHES (Int. Cl.4) |
| A | GB-A-2 091 757 (SHELL)<br>--- | | C 01 G<br>B 01 J |
| A | FR-A-2 554 433 (SHELL)<br>----- | | |

Le présent rapport a été établi pour toutes les revendications

| Lieu de la recherche | Date d'achèvement de la recherche | Examinateur |
|---|---|---|
| LA HAYE | 23-03-1989 | LIBBERECHT-VERBEECK E.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)